Europäisches Patentamt
European Patent Office
Office européen des brevets

(19) (11) Publication number: 0 298 601 A1

(12) **EUROPEAN PATENT APPLICATION**

(21) Application number: 88305144.3

(22) Date of filing: 06.06.88

(51) Int. Cl.4: C07D 473/32 , C07D 239/28

The title of the invention has been amended (Guidelines for Examination in the EPO, A-III, 7.3).

(30) Priority: 11.06.87 GB 8713695

(43) Date of publication of application: **11.01.89 Bulletin 89/02**

(84) Designated Contracting States: **AT BE CH DE ES FR GB GR IT LI LU NL SE**

(71) Applicant: **BEECHAM GROUP PLC**
**Beecham House Great West Road**
**Brentford Middlesex TW8 9BD(GB)**

(72) Inventor: **Wyatt, Paul Graham Beecham Pharmaceuticals**
**Great Burgh Yew Tree Bottom Road**
**Epsom Surrey KT18 5XQ(GB)**

(74) Representative: **Jones, Pauline et al**
**Beecham Pharmaceuticals Patents & Trade Marks Dept. Great Burgh Yew Tree Bottom Road**
**Epsom Surrey KT18 5XQ(GB)**

(54) **6-Deoxy guanine derivatives and pyrimidine intermediates.**

(57) A process for the preparation of a compound of formula (I) or a pharmaceutically acceptable salt thereof:

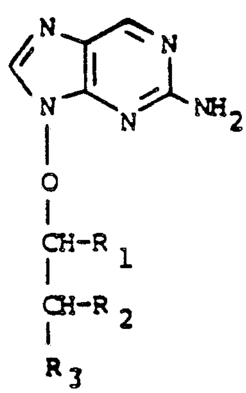

(I)

wherein $R_1$, $R_2$ and $R_3$ are as defined in formula (A); and in which any OH groups in $R_1$, $R_2$ and/or $R_3$ may be in the form of O-acyl, phosphate, cyclic acetal or cyclic carbonate derivatives thereof;
which process comprises the ring closure of a compound of formula (II):

EP 0 298 601 A1

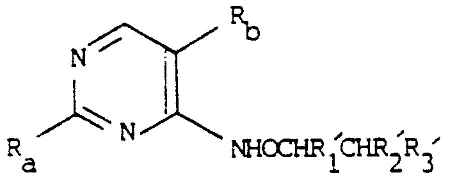

(II)

wherein $R_a$ is amino or a group or atom convertible thereto; $R_b$ is amino or an amino derivative and $R_1'$, $R_2'$ and $R_3'$ are $R_1$, $R_2$ and $R_3$ respectively or $R_1$, $R_2$, and/or $R_3$ wherein OH group(s) is/are protected; and thereafter converting Ra when other than amino, to amino; $R_1'$ to $R_1$, $R_2'$ to $R_2$ and/or $R_3'$ to $R_3$ when necessary and/or optionally forming a pharmaceutically acceptable salt or derivative thereof as defined.

## NOVEL PROCESS

This invention relates to a novel process for the preparation of 6-deoxy guanine derivatives, useful as antiviral agents.

EP-A-242482, the disclosure of which is incorporated herein by reference, discloses compounds of formula (A) and pharmaceutically acceptable salts thereof:

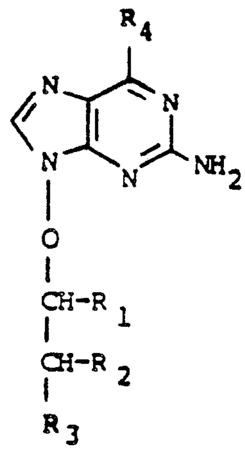

(A)

wherein

$R_1$ is hydrogen or $CH_2OH$;

$R_2$ is hydogen or, (when $R_1$ is hydrogen), hydroxy or $CH_2OH$;

$R_3$ is $CH_2OH$ or, (when $R_1$ and $R_2$ are both hydrogen), $CH(OH)CH_2OH$;

$R_4$ is hydrogen, hydroxy, amino or $OR_5$

wherein

$R_5$ is $C_{1-6}$ alkyl, phenyl or phenyl $C_{1-2}$ alkyl either of which phenyl moieties may be substituted by one or two halo, $C_{1-4}$ alkyl or $C_{1-4}$ alkoxy groups; and in which any OH groups in $R_1$, $R_2$ and/or $R_3$ may be in the form of O-acyl, phosphate, cyclic acetal or cyclic carbonate derivatives thereof.

There are groups of compounds within formula (A) as follows:

(a) $R_1$ and $R_2$ are both hydrogen and $R_3$ is $CH_2OH$, and derivatives thereof as defined;

(b) $R_1$ is hydrogen and $R_2$ and $R_3$ are both $CH_2OH$, and derivatives thereof as defined;

(c) $R_1$ is hydrogen, $R_2$ is hydroxy and $R_3$ is $CH_2OH$, and derivatives thereof as defined;

(d) $R_1$ is $CH_2OH$, $R_2$ is hydrogen and $R_3$ is $CH_2OH$, and derivatives thereof as defined.

(e) $R_1$ and $R_2$ are both hydrogen and $R_3$ is $CH(OH)CH_2OH$, and derivatives thereof as defined.

Examples of $R_5$ include methyl, ethyl, n- and iso-propyl, phenyl and benzyl optionally substituted by one or two or methyl, ethyl, n- and iso-propyl, methoxy, ethoxy, n- and iso- propoxy, fluoro, chloro, bromo or $CF_3$.

O-Acyl derivatives are normally those wherein one or two OH groups in $R_1$, $R_2$ and/or $R_3$ form carboxylic ester groups; such as $C_{1-7}$ alkanoyl and benzoyl optionally substituted by one or two $C_{1-4}$ alkyl, $C_{1-4}$ alkoxy, halogen or $CF_3$ groups. Preferably, carboxylic ester groups are $C_{1-7}$ alkanoyl groups, such as acetyl, propionyl, butyryl, heptanoyl and hexanoyl, most preferably acetyl or propionyl.

Examples of phosphate esters of the compounds of formula (A) include those where one of the acyclic -OH groups is replaced by $(HO)_2$-$PO_2$- groups or salts thereof, or where two -OH groups on carbon atoms are replaced by a bridging -O-P(OH)$O_2$- group.

When $R_1$, $R_2$ and $R_3$ together contain more than one OH group, cyclic acetal groups, such as -O-C-($C_{1-3}$alkyl)$_2$ -O- or cyclic carbonate, such as -O-CO-O- may be formed.

Examples of pharmaceutically acceptable salts of the compound of formula (A) are acid addition salts formed with a pharmaceutically acceptable acid such as hydrochloric acid, orthophosphoric acid and sulphuric acid. Pharmaceutically acceptable salts also include those formed with organic bases, preferably with amines, such as ethanolamines or diamines; and alkali metals, such as sodium and potassium.

When the compound of formula (A) contains a phosphate group suitable salts include metal salts, such as alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or

tris-(2-hydroxyethyl)-amine.

It will be appreciated that some of the compounds of formula (A) have at least one asymmetric centre, and therefore are capable of existing in more than one stereoisomeric form. The products of the process of the invention include each of these forms individually and mixtures thereof, including racemates. The isomers may be separated conventionally by chromatographic methods or using a resolving agent. Alternatively, the individual isomers may be prepared by asymmetric synthesis using chiral intermediates.

It will be further appreciated that, when $R_4$ is hydroxy in formula (A), the compound exists in the preferred tautomeric form of formula (B):

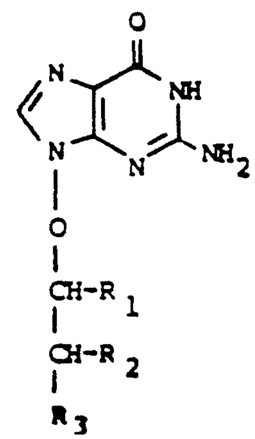

(B)

The compounds of formula (A) including their alkali metal salts may form solvates such as hydrates and these are included wherever a compound of formula (A) or a salt thereof is herein referred to.

It will also be appreciated that compounds of formula (A) wherein $R_4$ is other than hydroxy are prodrugs for the compounds of formula (A) wherein $R_4$ is hydroxy.

The compounds are described as useful in the treatment of infections caused by viruses, especially herpesviruses such as herpes simplex type 1, herpes simplex type 2; varicella zoster viruses; and also lentiviruses such as visna virus and human immunodeficiency virus.

Two alternative processes are described for the preparation of compounds wherein $R_4$ is hydrogen, both involving, as a final step, the conversion of a corresponding compound of formula (A) wherein $R_4$ is chloro, to $R_4$ is hydrogen, by means of a catalytic reduction as described by T.A. Krenitsky et. al. Proc. Natl. Acad. Sci. USA. 81, 3209 (1984).

This method has also been used for the preparation of other deoxy guanine derivatives, such as those described in EP-A-182024 and EP-A-186640.

A new process has now been discovered which obviates the need for reduction of a 6-chloropurine derivative and results in an overall improved yield.

Accordingly, the present invention provides a process for the preparation of a compound of formula (I) or a pharmaceutically acceptable salt thereof:

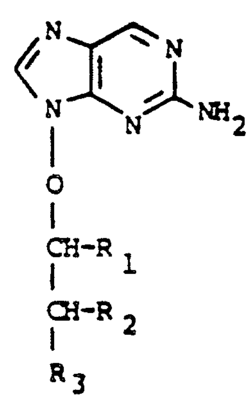

(I)

wherein $R_1$, $R_2$ and $R_3$ are as defined in formula (A); and in which any OH groups in $R_1$, $R_2$ and/or $R_3$ may

be in the form of O-acyl, phosphate, cyclic acetal or cyclic carbonate derivatives thereof; which process comprises the ring closure of a compound of formula (II):

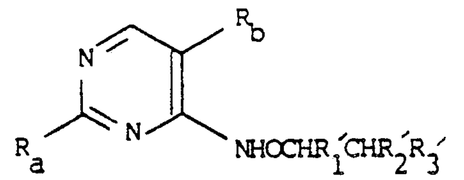

(II)

wherein $R_a$ is amino or a group or atom convertible thereto; $R_b$ is amino or an amino derivative and $R_1'$, $R_2'$ and $R_3'$ are $R_1$, $R_2$ and $R_3$ respectively or $R_1$ $R_2$, and/or $R_3$ wherein OH group(s) is/are protected; and thereafter converting $R_a$ when other than amino, to amino; $R_1'$ to $R_1$, $R_2'$ to $R_2$ and/or $R_3'$ to $R_3$ when necessary and/or optionally forming a pharmaceutically acceptable salt or derivative thereof as defined.

The ring closure may take place using a suitable cyclisation condensing agent such as diethoxymethylacetate or triethylorthoformate or by fusion. These methods are generally described in 'Comprehensive Organic Chemistry' p499-504, 1979, Vol 4. D.H. R. Barton and W.D. Ollis eds.).

Suitable values for $R_a$ include chloro, amino and formylamino. $R_a$ when chloro may be converted to amino by conventional methods, such as those described in EP-A-242482 for conversion of $R_4$ is chloro to $R_4$ is amino, or using $(CH_3)_3SiNH_2$ in toluene. $R_a$ is formylamino may beconverted to amino by conventional methods, such as that using aqueous hydrazine in ethanol at reflux temperatures.

Preferably $R_b$ is formylamino; although it may also be amino or ortho ester amino, such as $-NH-CH-(OC_2H_5)_2$.

Suitable values for protected OH groups in $R_1'$, $R_2'$ and $R_3'$ and methods for deprotection of protected OH groups in $R_1'$, $R_2'$ and $R_3'$ and methods for deprotection and formation of salts and derivatives are as described in EP-A-242482.

Compounds of the formula (II) may be prepared by reaction of a compound of formula (III):

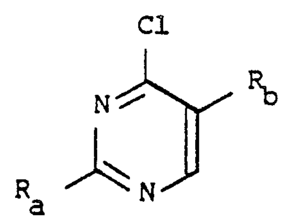

(III)

with a compound of formula (IV):

$R_3'CHR_2'CHR_1'ONH_2$ (IV)

This reaction is carried out in accordance with the procedures described hereinafter, in an inert solvent, such as 2-methoxyethyl ether at elevated temperatures, around 100° C, in the presence of an acid acceptor, such as diisopropylethylamine.

Intermediates of formula (III) are known or prepared by analogous methods. For example, the compound of formula (III) wherein $R_a$ is chloro and $R_b$ is amino may be prepared as follows:

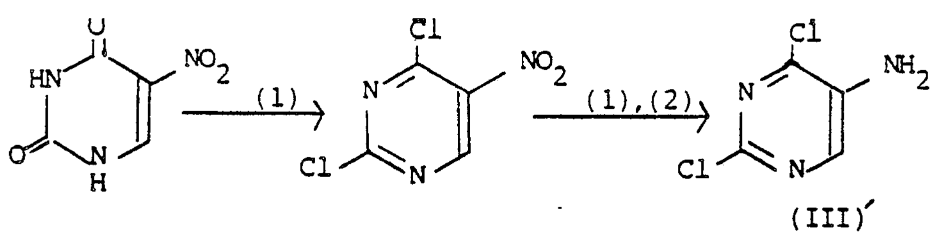


(III)'

(1) Whittaker. J. Chem. Soc (1951). 1565.

(2)(a) Inove. Chem. Pharm. Bull (Japan) 6, 343, (1958).

(2)(b) Inove. Chem. Pharm. Bull (Japan) 6, 349, (1958).

The compound of formula (III) wherein $R_a$ is amino and $R_b$ is amino may be prepared as follows:

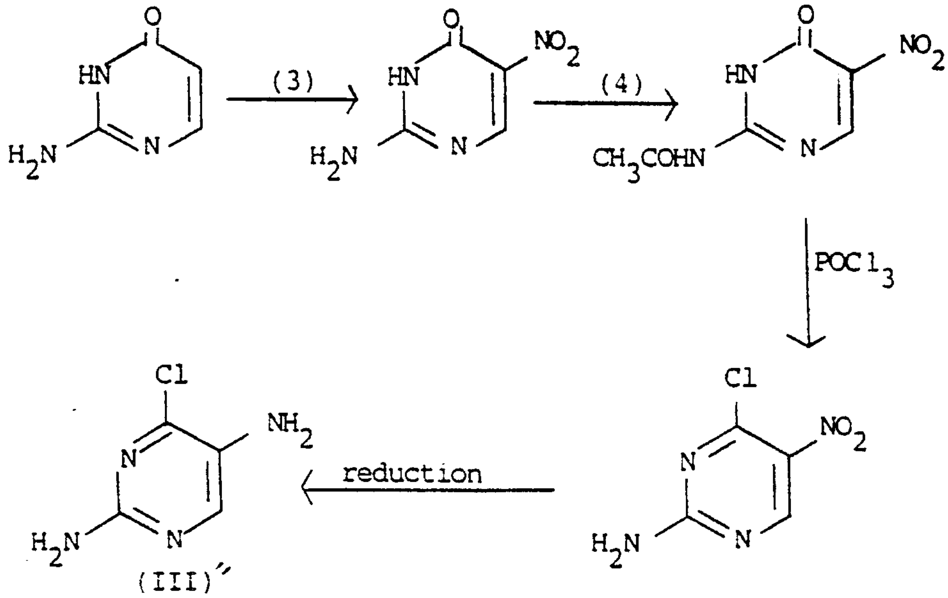


(III)

(3) Johns Am. Chem. J. 34, 554, (1905).

(4) Tong, Lee and Goodman, J.A.C.S. 86, 5664, (1964).

Compounds of the formula (IV) may be prepared as described in EP-A-242482.

Intermediates of the formula (II) are novel and form an aspect of the invention.

The process of the invention may be used to prepare the compounds of formula (I) which are the compounds of Examples 7, 9, 10, 12, 13, 16-19, 22, 23, 24, 29 and 30 of EP-A-242482.

The following Example illustrates the invention. The compound of the Example may be converted to 2-amino-9-(3-hydroxy-2-hydroxymethylprop-l-oxy)purine and derivatives thereof, according to the methods of examples 16-19 of EP-A-242482.

## Example

Preparation of 2-Amino-9-(2,2-dimethyl-1,3-dioxan-5-yloxy)purine

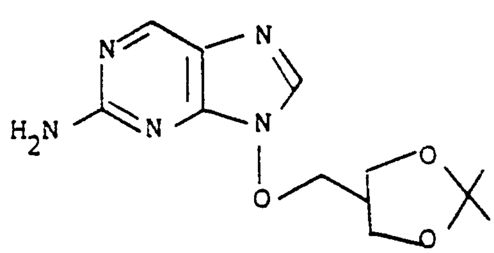

(a) 2.4-Dichloro-5-formamidopyrimidine

Acetic anhydride (12ml) was added to a mixture of 5-amino-2,4-dichloropyrimidine (2.34g, 14.4mmol) and formic acid (30ml) at 0° C. The mixture was stirred at 20° C for 4 hours, evaporated to dryness and co-evaporated with toluene. The crystalline product was homogeneous on t.l.c. (chloroform-methanol, 15.1) and used without further purification. (2.74g, 100%). IR: $\nu$max (KBr) 3445, 3350, 3240, 1635, 1570, 1515, 1415 $cm^{-1}$. $^1H$ NMR $\delta_H$ [$(CD_3)_2SO$] 8.45 (1H, s, H.2), 9.40 (1H,s,CH), 10.40 (1H,br.s, NH).

(b) 2-Chloro-4-(2,2-dimethyl-1,3-dioxan-5-ylmethoxyamino)-5-formamidopyrimidine

A mixture of 2,4-dichloro-5-formamidopyrimidine (2.5g, 13.0mmol), 2,2-dimethyl-1,3-dioxan-5-yl-methoxyamine (13.6mmol), diisopropylethylamine (4.5ml, 25.8mmol) and 2-methoxyethyl ether (50ml) was heated at 100° C for 4.5 hours. The suspension was cooled and evaporated under reduced pressure. The residue was chromatographed on silica gel (eluted with chloroform-methanol, 20:1), yielding the title compound (3.45g, 84%). $^1H$ NMR $\delta_H$ ($CDCl_3$) 1.45 (6H,s,2x$CH_3$), 2.05(1H m,CH), 3.80 (6H,m,3x$CH_2$), 8.3(1H,br,s,NH), 8.45 (1H,s,H-6).

(c) 2-Chloro-9(2,2-dimethyl-1,3-dioxan-5-ylmethoxy)purine

2-Chloro-4-(2,2-dimethyl-1,3-dioxan-5-ylmethoxyamino)-5-formamidopyrimidine (3.45g, 10.9mmol) in diethoxymethylacetate (100ml) was heated at 120° C for 2 hours. The mixture was then cooled and evaporated to a syrup. The residue was dissolved in methanol (50ml) and concentrated aqueous ammonia (2.5ml). The solution was then stirred at 20° C for 1 hour, evaporated under reduced pressure and the residue co-evaporated with toluene. Column chromatography on silica gel (eluted with chloroform-methanol, 60:1) gave the title compound (2.27g, 70%). IR: $\nu$max (KBr) 3117, 2993, 1599, 1571, 1339 $cm^{-1}$. $^1H$ NMR: $\delta_H$ [$(CD_3)_2SO$] 1.33 (3H,s,$CH_3$), 1.37 (3H,s,CH3), 2.08 (1H,m,CH), 3.81 (2H,d.d, J=5.8, 12.1Hz, 2xH(ax)), 4.04 (2H, d.d, J = 4.0, 12.0 Hz, 2xH (eq)), 4.52 (2H, d, J = 3.3Hz, $CH_2ON$), 9.00 (1H,s,H-8), 9.14(1H,s,H-6). Found: C, 48.34; H, 5.13; N 18.66%: $C_{12}H_{15}ClN_4O_3$ requires: C, 48.24; H, 5.07; N, 18.76%.

(d) 2-Amino-9-(2,2-dimethyl-1,3-dioxan-5-yloxy)purine

Method 1

A mixture of 2-chloro-9-(2,2-dimethyl-1,3-dioxan-5-yloxy)purine (100mg, 0.335mmol) and liquid ammonia (20ml) was left in an autoclave at 20° C for 48 hours. The solution was evaporated using a stream of nitrogen and the residue chromatographed on silica gel (eluted with chloroform-methanol, 15:1) affording the title compound (57.5mg, 62%). IR: $\nu$max (KBr) 3336, 3203, 1647, 1618, 1578, 1430$cm^{-1}$. $^1H$ NMR: $\delta_H$ [$(CD_3)_2SO$] 1.97 (1H,m,CH), 3.55 (4H,m, 2x$CH_2OH$), 4.33 (2H,d, J=6.3Hz $CH_2ON$), 4.59 (2H,t, J=5.3HZ, $D_2O$ exchangeable, 2xOH), 6.70 (2H, br.s, $D_2O$ exchangeable, $NH_2$), 8.30 (1H,s,H-8), 8.59 (1H,s,H-6).

A subsequent reaction indicated that this reaction time is not needed; about 7 hours should be sufficient.

Method 2

A stream of ammonia was bubbled through a solution of 2-chloro-9-(2,2-dimethyl-1,3-dioxan-5-ylmethoxy)purine (100mg) in dimethylsulphoxide at 100° C. After 3 hours the solution was cooled, evaporated to dryness and the residue purified by column chromatography on silica gel eluting with chlorform-methanol (15:1) to afford 2-amino-9-(2,2-dimethyl-1,3-dioxan-5-ylmethoxy) purine (55mg; 59%): IR: $\nu_{max}$ (KBr) 3327, 3193, 1655, 1622, 1580, 1515 and 1434 $cm^{-1}$. $^1H$ NMR: $\delta_H$ [$(CD_3)_2SO$] 1.33 (3H,s,$CH_3$), 1.35 (3H,s,$CH_3$), 2.02 (1H,m,CH), 3.79 (2H,dd, J=12.1 Hz, and 5.8 Hz, 2x$H_{(ax)}$), 4.05 (2H, dd, J=12.1 Hz and 4.1 Hz, 2xH $_{(eg)}$.), 4.39 (2H,d, J=7.1HZ, $CH_2ON$),6.70 (2H,s,$D_2O$ exchangeable, $NH_2$), 8.34 (1H,s,H-8), 8.59 (1H,s,H-6).

## Claims

1. A process for the preparation of a compound of formula (I) or a pharmaceutically acceptable salt thereof:

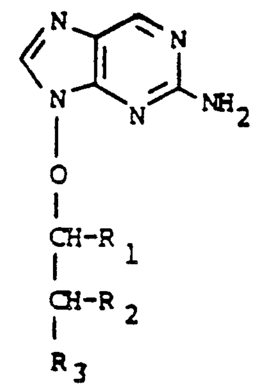

(I)

wherein $R_1$, $R_2$ and $R_3$ are as defined in formula (A); and in which any OH groups in $R_1$, $R_2$ and/or $R_3$ may be in the form of O-acyl, phosphate, cyclic acetal or cyclic carbonate derivatives thereof,
which process comprises the ring closure of a compound of formula (II):

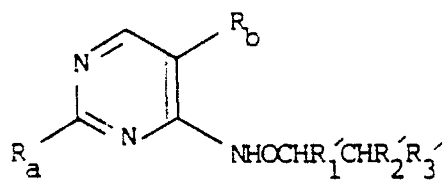

(II)

wherein $R_a$ is amino or a group or atom convertible thereto; $R_b$ is amino or an amino derivative and $R_1'$, $R_2'$ and $R_3'$ are $R_1$, $R_2$ and $R_3$ respectively or $R_1$, $R_2$, and/or $R_3$ wherein OH group(s) is/are protected; and thereafter converting $R_a$ when other than amino, to amino; $R_1'$ to $R_1$, $R_2'$ to $R_2$ and/or $R_3'$ to $R_3$ when necessary and/or optionally forming a pharmaceutically acceptable salt or derivative thereof as defined.

2. A process according to claim 1 for the preparation of a compound of formula (I) as defined in claim 1, wherein $R_1$ is hydrogen and $R_2$ and $R_3$ are both $CH_2OH$, and derivatives thereof as defined in claim 1.

3. A process according to claim 1 for the preparation of a compound of formula (I) as defined in claim 1, wherein $R_1$ is hydrogen, $R_2$ is hydroxy and $R_3$ is $CH_2OH$, and derivatives thereof as defined in claim 1.

4. A process according to claim 1 for the preparation of a compound of formula (I) as defined in claim 1, wherein $R_1$ is $CH_2OH$, $R_2$ is hydrogen and $R_3$ is $CH_2OH$, and derivatives thereof as defined in claim 1.

5. A process according to claim 1 for the preparation of a compound of formula (I) as defined in claim 1, wherein $R_1$ and $R_2$ are both hydrogen and $R_3$ is $CH(OH)CH_2OH$, and derivatives thereof as defined in claim 1.

6. A process according to claim 1 for the preparation of a compound or formula (I) as defined in any one of claims 2 to 5 wherein OH groups in $R_1$, $R_2$, and/or $R_3$ are in the form of an acyl derivative thereof wherein the acyl derivative(s) is/are an acetate, hexanoate or benzoate.

7. A process according to claim 1 for the preparation of a compound of formula (I) which is:
2-amino-9-(2,3-dihydroxyprop-1-oxy)purine,
2-amino-9-(1,4-dihydroxybut-2-oxy)purine,
2-amino-9-(3-hydroxyprop-1-oxy)purine,
2-amino-9-(1,4-diacetoxybut-2-oxy)purine,
2-amino-9-(1,4-dibutyryloxybut-2-oxy)purine,
2-amino-9-(3-hydroxy-2-hydroxymethylprop-1-oxy)purine,
2-amino-9-(3-acetoxy-2-acetoxymethylprop-1-oxy)purine,

2-amino-9-(3-propionyloxy-2-propionyloxymethylprop-1-oxy)purine,
2-amino-9-(3-benzoyloxy-2-benzoyloxymethylprop-1-oxy)purine,
2-amino-9-(3-acetoxyprop-1-oxy)purine,
2-amino-9-(3-hexanoyloxyprop-1-oxy)purine,
2-amino-9-(3-benzoyloxyprop-1-oxy)purine,
2-amino-9-(3,4-dihydroxybut-1-oxy)purine,
(R)-2-amino-9-(2,3-dihydroxyprop-1-oxy)purine or
(S)-2-amino-9-(2,3-dihydroxyprop-1-oxy)purine.

8. 2-Amino-9-(3-hydroxy-2-hydroxymethylprop-1-oxy)purine whenever prepared by a process according to claim 1, or an obvious chemical equivalent.

9. An intermediate of formula (II) as defined in claim 1.

European Patent Office

# EUROPEAN SEARCH REPORT

Application number: EP 88305144.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,D, A | EP - A2 - 0 242 482 (BEECHAM GROUP PLC)<br>* Claims 1,11; examples *<br>-- | 1-8 | C 07 D 473/32<br>C 07 D 239/28 |
| D,A | EP - A1 - 0 186 640 (ASTRA LAKEMEDEL AKTIEBOLAG)<br>* Pages 13,14 *<br>-- | 1,9 | |
| A | EP - A1 - 0 158 847 (THE WELLCOME FOUNDATION LIMITED)<br>* Claim 7d); page 13, last paragraph; page 14, formula and first paragraph *<br>---- | 1,9 | |

TECHNICAL FIELDS SEARCHED (Int. Cl.4)

C 07 D 473/00
C 07 D 239/00

The present search report has been drawn up for all claims

| Place of search | Date of completion of the search | Examiner |
|---|---|---|
| VIENNA | 22-08-1988 | HERING |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document